# EUROPÄISCHE PATENTSCHRIFT

(11) **EP 2 681 185 B1**
(45) Veröffentlichungstag und Bekanntmachung des Hinweises auf die Patenterteilung: **11.07.2018**
(21) Anmeldenummer: 12706245.3
(22) Anmeldetag: 27.02.2012
(51) Int. Cl.: C07C 205/56, C07C 253/30, C07C 319/20, C07C 67/343, C07C 333/24, C07C 69/612, C07C 69/734, C07C 69/616, C07C 69/75, C07C 69/76

(54) **VERFAHREN ZUR HERSTELLUNG VON ARYL- UND HETEROARYLESSIGSÄURE-DERIVATEN**
PROCESS FOR PREPARING ARYL- AND HETEROARYLACETIC ACID DERIVATIVES
PROCÉDÉ DE PRODUCTION DE DÉRIVÉS D'ACIDES ARYL- ET HÉTÉROARYLACÉTIQUE

(30) Priorität: 02.03.2011 US 201161448379 P; 02.03.2011 EP 11156559
(43) Veröffentlichungstag der Anmeldung: 08.01.2014
(60) Teilanmeldung: 18162783.7
(73) Patentinhaber: Bayer Intellectual Property GmbH, 40789 Monheim am Rhein (DE)
(72) Erfinder: HIMMLER, Thomas, 51519 Odenthal (DE); GOOSSEN, Lukas J., 67663 Kaiserslautern (DE); RUDOLPHI, Felix, 69198 Schriesheim (DE); SONG, Bingrui, 67655 Kaiserslautern (DE)
(74) Vertreter: BIP Patents
(86) Internationale Anmeldenummer: PCT/EP2012/053233
(87) Internationale Veröffentlichungsnummer: WO 2012/116942

(56) Entgegenhaltungen:
- WO-A1-2009/121919
- YOSHINORI KONDO ET AL.: "Palladium catalyzed arylation of malonate accompanying in situ dealkoxycarbonylation", CHEMICAL COMMUNICATIONS, 2001, Seiten 2704-2705, XP002630825, ISSN: 1359-7345 in der Anmeldung erwähnt
- F.A. Carey; R.J. Sundberg: "Organische Chemie Ein weiterführendes Lehrbuch", 1995, VCH Verlagsgesellschaft mbH, XP002671765, ISBN: 3-527-29217-9 Seiten 876-877, Seite 876, Absatz 2 Seite 877, letzter Absatz
- SONG, B. ET AL.: "Practical Synthesis of 2-Arylacetic Acid Esters via Palladium-Catalyzed Dealkoxycarbonylative Coupling of Malonates with Aryl Halides", ADV.SYNTH.CATAL., vol. 353, 2011, pages 1565-1574,

## Beschreibung

Die Erfindung betrifft ein Verfahren zur Herstellung von Aryl- und Heteroarylessigsäuren und ihrer Derivate durch Umsetzung von Aryl- oder Heteroarylhalogeniden mit Malonsäurediestern in Gegenwart eines Palladiumkatalysators, einer oder mehrerer Basen und gegebenenfalls eines Phasentransferkatalysators. Dieses Verfahren ermöglicht die Darstellung einer Vielzahl funktionalisierter Aryl- und Heteroarylessigsäuren und ihrer Derivate, insbesonders auch die Herstellung von Arylessigsäuren mit sterisch anspruchsvollen Substituenten.

Üblicherweise werden Phenylessigsäure-Derivate in mehrstufigen Synthesen hergestellt, die meist eine geringe Gruppentoleranz aufweisen. Die Herstellung kann beispielsweise ausgehend von Acetophenonen durch eine Willgerodt-Kindler-Reaktion erfolgen (siehe beispielsweise H.E. Zaugg et al., J. Amer. Chem. Soc. 70 (1948) 3224-8*).* Bei dieser Methode fallen jedoch große Mengen schwefelhaltiger Abfälle an. Außerdem können stark geruchsbelästigende leichtflüchtige Schwefelverbindungen auftreten.

Eine weitere Methode zur Herstellung von Arylessigsäuren geht von Benzylbromiden oder -chloriden aus. Man stellt daraus z.B. mit Natriumcyanid die entsprechenden Nitrile her, welche anschließend verseift werden. Die benötigten Benzylbromide oder -chloride können beispielsweise durch Brom- oder Chlormethylierung der entsprechenden Aromaten erhalten werden. Hierbei ist allerdings nachteilig, dass das Auftreten stark kanzerogener Verbindungen wie Bis(chlormethyl)ether oder Bis-(brommethyl)ether nicht ausgeschlossen werden kann, so dass technisch ein hoher Sicherheitsaufwand betrieben werden muss. Außerdem führt die Halogenmethylierung von substituierten Aromaten in vielen Fällen zu Isomerengemischen.

Die Carbonylierung von Benzylhalogeniden in Gegenwart von Alkoholen liefert ebenfalls Phenylessigsäureester. Die bereits genannte begrenzte Verfügbarkeit von Benzylhalogeniden und die Notwendigkeit, toxisches CO-Gas einzusetzen, gegebenenfalls auch unter erhöhtem Druck, sind weitere Nachteile dieses Verfahrens.

Es ist auch bereits bekannt geworden, α-Chlor-acetophenone zu ketalisieren und die Ketale dann einer Umlagerungsreaktion zu unterwerfen (C.Giordano et al., Angew. Chem. 96 (1984) 413-9)*.* Die α-Chlor-acetophenone erhält man entweder durch Chlorierung von Acetophenonen oder direkt durch eine Friedel-Crafts-Acylierung des betreffenden Aromaten mit Chloracetylchlorid. Damit ergibt sich wieder der Nachteil, dass die Friedel-Crafts-Acylierungen an substituierten Aromaten häufig unselektiv verlaufen.

Eine weitere bekannte Methode zur Herstellung von Phenylessigsäuren besteht darin, ein entsprechendes Anilin im ersten Schritt zu diazotieren, die erhaltene Diazoniumverbindung im zweiten Schritt mit Vinylidenchlorid umzusetzen, und die so erhaltene Trichlor- oder Brom-dichlorethylverbindung dann im dritten Schritt mit Wasser oder Alkoholen zur Arylessigsäure bzw. deren Estern umzusetzen (siehe beispielsweise V.M.Naidan und A.V.Dombrovskii, Zhurnal Obshchei Khimii 34 (1984)1469-73*;* EP-A-835243*).* Diese Reaktion liefert in der Regel jedoch nur mit solchen Anilinen gute Ausbeuten, die elektronenziehende Reste am Aromaten tragen und in denen die Aminogruppe nicht sterisch blockiert ist.

Weiterhin bekannt ist die Umsetzung von Brombenzolen mit Chloressigsäurederivaten in Gegenwart von stöchiometrischen Mengen Silber oder Kupfer bei 180 - 200 °C. Nachteilig an diesen Verfahren ist die hohe Temperatur, die eine Anwendung bei temperaturempfindlichen Verbindungen ausschließt, die geringe Ausbeute und die Anwendung stöchiometrischer Mengen teurer und schwer wieder aufzuarbeitender Metalle.

Die Umsetzung von Aryl-Grignardverbindungen mit α-Halogenessigsäure-Derivaten führt ebenfalls zu Phenylessigsäurederivaten. Nachteilig ist allerdings die durch die Verwendung von schwer zu handhabenden, hochreaktiven Grignardverbindungen äußerst eingeschränkte Toleranz funktioneller Gruppen.

Alternativ zu den genannten Verfahren wurden auch Kreuzkupplungen von Arylhalogeniden mit Reformatsky-Reagenzien, Zinn-, Kupfer- und anderen Enolaten oder Ketenacetalen beschrieben (siehe z. B. J. Am. Chem. Soc. 1959, 81, 1627-1630*;* J. Organomet. Chem. 1979, 177, 273-281*;* Synth. Comm. 1987, 17, 1389-1402*;* Bull. Chem. Soc. Jpn. 1985, 58, 3383-3384*;* J. Org. Chem. 1993, 58, 7606-7607*;* J. Chem. Soc. Perkin 1 1993, 2433-2440*;* J. Am. Chem. Soc. 1975, 97, 2507-2517*;* J. Am. Chem. Soc. 1977, 99, 4833-4835*;* J. Am. Chem. Soc. 1999, 121, 1473-78*;* J. Org. Chem. 1991, 56, 261-263*,* Heterocycles 1993, 36, 2509-2512*,* Tetrahedron Lett. 1998, 39, 8807-8810). Diese Verfahren sind allerdings in ihrer Anwendbarkeit limitiert. So sind Reformatzky-Reagenzien und Ketenacetale aufwendig in der Darstellung und Handhabung. Die Verwendung von Zinnverbindungen ist aus toxikologischen Gründen nachteilig und der Einsatz von stöchiometrischen Mengen Kupfer verursacht erhebliche Kosten bei der Entsorgung. Die Verwendung von Enolaten ist in der Regel nur möglich, wenn keine weiteren enolisierbaren Gruppen im Molekül vorhanden sind. Beispielsweise sind Ketone daher als Substrate für derartige Verfahren ausgeschlossen. Einige elektrochemische Prozesse sind ebenfalls bekannt (Synthesis 1990, 369-381*;* J. Org. Chem. 1996, 61, 1748-1755), allerdings sind diese Verfahren aufgrund der aufwendigen Reaktionsführung und der geringen Raum-Zeit-Ausbeuten nachteilig.

Ebenfalls bereits bekannt ist eine Methode zur Herstellung von Phenylessigsäure-Derivaten durch eine Palladium-katalysierte Kupplungsreaktion zwischen Arylboronsäuren und Ethylbromacetat *(*Chem.Commun. 2001, 660-70*;* DE-A- 10111262). Dieses Verfahren erfordert jedoch die Herstellung der Boronsäuren, üblicherweise aus den entsprechenden Aryl- oder Heteroarylhalogeniden. Außerdem konnte dieses Verfahren bisher nicht zur Herstellung von sterisch anspruchsvollen, beispielsweise 2,6-disubstituierten Phenylessigsäure-Derivaten verwendet werden. In Chem. Commun. 2001, 660-70 wird zwar angegeben, dass auch sterisch gehinderte Arylboronsäuren unter den dort beschriebenen Bedingungen gut umgesetzt werden können. Die Beispiele enthalten jedoch nur die 2-Tolylboronsäure als sterisch gehindertes Substrat. Stärker sterisch eingeschränkte Arylboronsäuren wie z.B. 2,6-Dialkylphenyl-boronsäuren werden nicht beschrieben.

Eine weitere bekannte Methode besteht in der Palladium- oder Kupfer-katalysierten Kupplungsreaktion von Arylhalogeniden mit Malonestern oder β-Ketoestern, gefolgt von einer thermisch induzierten Dealkoxycarbonylierung oder retro-Claisenkondensation. Dabei wurden Aryliodide und aktivierte Arylbromide mit Malonsäurediethylester in Gegenwart eines Palladium-Katalysators und 10 Äquivalenten sehr teuren Caesiumcarbonats umgesetzt, wobei Reaktionszeiten von bis zu 76 Stunden notwendig waren (Chem. Commun. 2001, 2704-2705). Höhere Ausbeuten bei kürzeren Reaktionszeiten sind möglich, erfordern allerdings den Einsatz sehr spezieller, nur aufwendig herzustellender N-heterocyclischen Carbenliganden; zudem wird auch hier das teure Cesiumcarbonat verwendet (Tetrahedron Lett. 2004, 45, 5823-5825). Die Palladium- oder Kupfer-katalysierte Arylierung von Acetessigestern, gefolgt von einer *in situ* Deacetylierung, hat letztendlich einen nur engen Anwendungsbereich; außerdem ist die Deacetylierung häufig nicht vollständig, woraus unbefriedigende Ausbeuten an Arylessigsäureestern resultieren (Tetrahedron Lett. 2004, 45, 4261-4264; Tetrahedron Lett. 2007, 48, 3289-3293).

In Chemical Communications 2001, Seiten 2704-2705 wird ein Verfahren zur Herstellung von Aryl- und Heteroarylessigsäureestern aus einer Aryl- bzw. Heteroarylhalogen-Verbindung und Diethylmalonat in Anwesenheit von Pd₂(dba)₃, tBu₃P, Cs₂CO₃ und DME offenbart, wobei kein Phasentransferkatalysator zum Einsatz kommt.

Alle bisher bekannt gewordenen Methoden zur Herstellung von Phenylessigsäure-Derivaten, insbesonders auch sterisch anspruchsvoll substituierten, weisen demnach z.T. erhebliche Mängel und Nachteile auf, die ihre Anwendung erschweren. Da allgemein Phenylessigsäuren, und unter ihnen gerade auch sterisch anspruchsvoll substituierte, wichtige Vorprodukte beispielsweise für Wirkstoffe im Pflanzenschutz sind, besteht Bedarf an einer technisch einfachen und hocheffizienten Methode zur Herstellung solcher Verbindungen.

Überraschenderweise wurde nun ein Verfahren zur Herstellung von Aryl- und Heteroarylessigsäuren und ihrer Derivate aus Aryl- und Heteroarylhalogeniden und Malonestern gefunden, welches dadurch gekennzeichnet ist, dass die Umsetzung in Gegenwart von einem Palladiumkatalysator, einem Phosphin, und
A) einer anorganischen Base und einem Phasentransferkatalysator
   durchgeführt wird, wobei sich nach der Kupplungsreaktion *in situ* eine Dealkoxycarbonylierung anschließt.

Die Entdeckung im Verfahrensschritt A), dass der Zusatz eines Phasentransferkatalysators die Selektivität der Umsetzung positiv beeinflusst, war nicht vorhersehbar und macht die Entdeckung dieses Verfahrens besonders überraschend. Durch den Einsatz des Phasentransferkatalysators ist es erstmals möglich, Selektivität und Ausbeute signifikant zu Gunsten des gewünschten Produktes zu verschieben. Dies macht das Verfahren deutlich wirtschaftlicher als die nach dem Stand der Technik bekannten Verfahren.

Das erfindungsgemäße Verfahren zur Herstellung von Aryl- und Heteroarylcarbonylverbindungen ist dadurch gekennzeichnet, dass man Aryl- oder Heteroarylhalogenide der Formel (I)

Ar-Hal (I)

in welcher
Hal für Chlor, Brom Iod steht und
Ar für die Gruppe 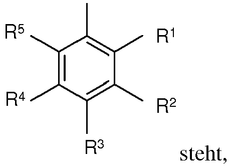 wobei
R¹, R², R³, R⁴ und R⁵ unabhängig voneinander gleich oder verschieden für Wasserstoff, Amino, Cyano, Nitro, Halogen, für gegebenenfalls durch Halogen substituiertes C₁-C₆-Alkyl, C₁-C₆-Thioalkyl, Thiophenyl, C₁-C₆-Alkoxy, C₆-C₁₀-Aryloxy, Phenyl, -CO-C₆-C₁₀-Aryl, -CO-C₁-C₃-Alkyl, - COO-C₁-C₆-Alkyl oder -COO-C₆-C₁₀-Aryl stehen,
der Rest Ar kann darüber hinaus auch für einen heteroaromatischen Rest wie 2-Pyridyl, 3-Pyridyl, 4-Pyridyl, 2-Furyl-, 3-Furyl, 2-Thienyl, oder 3-Thienyl stehen oder
der Rest Ar kann auch für 1- oder 2-Naphthyl stehen,
mit Malonsäureestern der Formel (II) 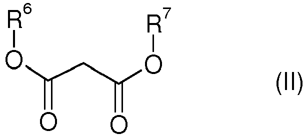 in welcher
   R⁶ und R⁷ unabhänging voneinander für gegebenenfalls substituiertes C₁-C₈-Alkyl, Phenyl, Aryl, oder für NR⁸R⁹ steht,
   wobei R⁸ und R⁹ unabhängig voneinander gleich oder verschieden für C₁-C₄-Alkyl oder für gegebenenfalls durch C₁-C₃-Alkyl, welches durch Fluor oder Chlor substituiert sein kann, durch Nitro, Cyano oder Di-C₁-C₃-Alkylamino substituiertes Phenyl stehen, oder gemeinsam mit dem Stickstoffatom, an das sie gebunden sind, für einen gesättigten oder ungesättigten, substituierten oder unsubstituierten Cyclus stehen,
   in Gegenwart eines Palladiumkatalysators, eines Phosphin-Liganden und
      A) einer anorganischen Base und eines Phasentransferkatalysators
         gegebenenfalls unter Verwendung eines organischen Lösungsmittels
         zu α-Arylmethyl-carbonylverbindungen der Formel (III) 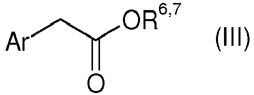 in welcher Ar und die Reste R⁶ bzw. R⁷ die oben angegebenen Bedeutungen haben umsetzt.

Dabei werden intermediär 2-Arylmalonsäurediester der Formel (IV) gebildet, aber nicht isoliert.

Diese Umsetzung wird demnach durch die folgende Reaktionsgleichung veranschaulicht:

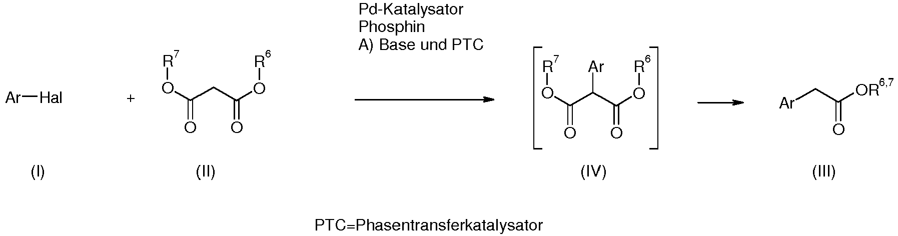

Bevorzugte Substituenten bzw. Bereiche der in der oben und nachstehend erwähnten Formeln aufgeführten Reste werden im Folgenden erläutert:
Ar steht bevorzugt für 1- oder 2-Naphthyl, 3-Thienyl oder für die Gruppe 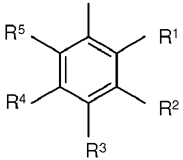 wobei
   R¹, R², R³, R⁴ und R⁵ stehen bevorzugt unabhängig voneinander gleich oder verschieden für Wasserstoff, Amino, Cyano, Nitro, Fluor, für gegebenenfalls durch Fluor substituiertes C₁-C₄-Alkyl, C₁-C₄-Thioalkyl, Thiophenyl, C₁-C₄-Alkoxy, C₆-C₁₀-Aryloxy, Phenyl, -CO-C₆-C₈-Aryl -CO-C₁-C₃-Alkyl, -COO-C₁-C₄-Alkyl oder -COO-C₆-C₈-Aryl stehen,
   Hal steht bevorzugt für Chlor, Brom oder Iod,
   R⁶ und R⁷ stehen bevorzugt unabhängig voneinander gleich oder verschieden für C₁-C₄-Alkyl.
Ar steht besonders bevorzugt für 1-oder 2-Naphthyl, 3-Thienyl oder für die Gruppe 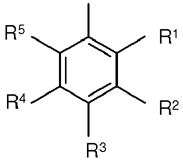 wobei
   R¹, R², R³, R⁴ und R⁵ stehen besonders bevorzugt unabhängig voneinander gleich oder verschieden für Wasserstoff, Amino, Cyano, Nitro, Fluor, Methyl, Methylthio, Ethyl, i-Propyl, n-Propyl, CF₃, C₂F₅, C₃F₇, Methoxy, Ethoxy, Phenyl, -CO-Phenyl, -CO-Methyl, -CO-Ethyl, -COO-Methyl, -COO-Ethyl oder -COO-Phenyl stehen,
   Hal steht besonders bevorzugt für Chlor, Brom oder Iod,
   R⁶ und R⁷ stehen besonders bevorzugt unabhängig voneinander für Methyl oder Ethyl, hervorgehoben für Ethyl.
   Ar steht ganz besonders bevorzugt für 1-Naphthyl, 2-Naphthyl, Phenyl, 4-N,N-Dimethylaminophenyl, 4-Methylthiophenyl, 4-Methoxyphenyl, 4-Ethoxyphenyl, 3-Methoxyphenyl, 2-Methoxyphenyl, 2-Methylphenyl, 3-Methylphenyl, 4-Methylphenyl, 4-Fluorphenyl, 3-Fluorphenyl, 2-Fluorphenyl, 2-Ethylphenyl, 4-Ethoxycarbonylphenyl, 3-Thienyl.
   Ar steht auch ganz besonders bevorzugt für 2,6-Dimethylphenyl, 2,4,6-Trimethylphenyl, 4-Cyanophenyl, 4-Cyano-2-methylphenyl, 3-Cyanophenyl, 4-Ethoxycarbonylphenyl, 4-Trifluormethylphenyl, 4-Acetylphenyl, 4-Nitrophenyl, 4-Benzoylphenyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Zwischenprodukte entsprechend.

Die Arylhalogenide der Formel (I) sind prinzipiell bekannt oder können nach bekannten Methoden hergestellt werden.

Die Verbindungen der Formel (II) sind prinzipiell bekannt oder können nach bekannten Methoden hergestellt werden.

Als Basen werden im erfindungsgemäßen Verfahrensschritt A) anorganische Basen wie Alkali- oder Erdalkalihydroxide, -carbonate, -bicarbonate, -oxide, -phosphate, -hydrogenphosphate, -fluoride oder -hydrogenfluoride eingesetzt. Vorzugsweise werden Alkali- und Erdalkaliphosphate, -carbonate oder -fluoride verwendet und besonders bevorzugt werden Natrium- und Kaliumphosphat verwendet. Hervorgehoben ist Kaliumphosphat.

Im erfindungsgemäßen Verfahren werden 1 bis 10 Äquivalente der jeweiligen Base eingesetzt. Vorzugsweise werden 1,2-5 Äquivalente der Base eingesetzt.

Als Palladium-Katalysatoren werden im erfindungsgemäßen Verfahren Palladium(II)-Salze wie etwa Palladiumchlorid, -bromid, -iodid, -acetat, -acetylacetonat, die wahlweise durch weitere Liganden wie z. B. Alkylnitrile stabilisiert sein können, bzw. Pd(0)-Spezies wie Palladium auf Aktivkohle, Pd(PPh₃)₄, Bis(dibenzylidenaceton)palladium oder Tris(dibenzylidenaceton)dipalladium eingesetzt. Bevorzugt sind Bis(dibenzylidenaceton)palladium, Tris(dibenzylidenaceton)dipalladium, Palladiumchlorid, Palladiumbromid und Palladiumacetat; hervorgehoben sind Bis(dibenzylidenaceton)palladium und Palladiumacetat.

Die Menge an im erfindungsgemäßen Verfahren eingesetzten Palladium-Katalysator beträgt 0,001 bis 5 Molprozent, bezogen auf eingesetztes Arylhalogenid. Bevorzugt werden 0,005 bis 3 Molprozent eingesetzt; besonders bevorzugt sind 0,01 bis 1 Molprozent.

Als Phosphinliganden werden im erfindungsgemäßen Verfahren Liganden PR¹⁰R¹¹R¹² eingesetzt, wobei die Reste R¹⁰, R¹¹ und R¹² für Wasserstoff, lineares und verzweigtes C₁-C₈-Alkyl, Vinyl-, Aryl-, oder Heteroaryl aus der Reihe Pyridin, Pyrimidin, Pyrrol, Thiophen oder Furan stehen, die ihrerseits mit weiteren Substituenten aus der Reihe lineares und verzweigtes C₁-C₈-Alkyl oder C₆-C₁₀-Aryl, lineares und verzweigtes C₁-C₈-Alkyloxy oder C₁-C₁₀-Aryloxy, halogeniertes lineares und verzweigtes C₁-C₈-Alkyl oder halogeniertes C₆-C₁₀-Aryl, lineares und verzweigtes C₁-C₈-Alkyl oder C₆-C₁₀-Aryloxycarbonyl, lineares und verzweigtes C₁-C₈-Alkylamino, lineares und verzweigtes C₁-C₈-Dialkylamino, C₁-C₈-Arylamino, C₁-C₈-Diarylamino, Formyl, Hydroxy, Carboxyl, Cyano, und Halogene wie F, Cl, Br und I substituiert sein können, in situ erzeugt.

Bevorzugte Phosphinliganden sind Trialkylphosphine wie Triethylphosphin, Tri-n-butyl-phosphin, Tri-tert-butyl-phosphin, Tricyclohexylphosphin, Tris(1-adamantyl)phosphin, n-Butyl-di(1-adamantyl)-phosphin (cataCXium® A), Benzyl-di(1-adamantyl)-phosphin (cataCXium® ABn), 2-(Di-tert-butylphosphino)biphenyl (JohnPhos), 2-(Di-cyclohexylphosphino)-2'-(N,N-dimethylamino)biphenyl (DavePhos) und 2-(Di-cyclohexylphosphino)-2',6'-dimethoxy-1,1'-biphenyl (SPhos). Besonders bevorzugt ist Tri-tert.butyl-phosphin.

Tri-tert.butyl-phosphin kann als freies Phosphin oder in Form des HBF₄-Adduktes eingesetzt werden.

Alternativ dazu können auch definierte Palladiumkomplexe eingesetzt werden, die aus den oben genannten Liganden in einem oder mehreren Verfahrensschritten zuvor erzeugt wurden.

Beim erfindungsgemäßen Verfahren werden 1 - 20 Moläquivalente Phosphin bezogen auf die eingesetzte Menge Palladium eingesetzt. Vorzugsweise werden 1 - 4 Moläquivalente eingesetzt.

Im erfindungsgemäßen Verfahrensschritt A) wird ein Phasentransferkatalysator aus der Reihe der quaternären Ammoniumsalze, der quaternären Phosphoniumsalze oder der Kronenether eingesetzt.

Die Phasentransferkatalysatoren aus der Reihe der quaternären Ammoniumsalze oder der quaternären Phosphoniumsalze besitzen vorzugsweise die Formel (V)

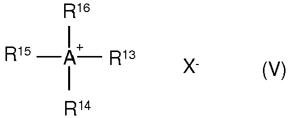

Die Reste R¹³, R¹⁴, R¹⁵ und R¹⁶ stehen unabhängig voneinander gleich oder verschieden für C₁-C₂₈-Alkyl, gegebenenfalls verzweigtes C₁-C₂₈-Alkyl, C₆-C₁₀-Aryl, oder Benzyl.

A steht für N oder P.

Der Rest X steht für Halogen, Hydrogensulfat, Sulfat, Dihydrogenphosphat, Hydrogenphosphat, Phosphat oder Acetat.

Bevorzugt steht X für Brom, Chlor, Fluor, Hydrogensulfat, Sulfat, Phosphat und Acetat.

Als solche Phasentransferkatalysatoren seien beispielhaft Tetrabutylammoniumfluorid, -chlorid, -bromid, -iodid, -acetat, Tetraethylammoniumiodid, Benzyltriethylammoniumbromid, Dodecyltrimethylammoniumbromid und Methyl-tridecylammoniumchlorid (Aliquat 336) genannt.

Die Phasentransferkatalysatoren aus der Reihe der Kronenether besitzen die Formel (VI) 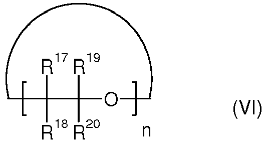 in der n für eine Zahl zwischen 4 und 8 steht
und die Reste
R¹⁷ bis R²⁰ unabhängig voneinander für Wasserstoff, C₁-C₄-Alkyl oder Phenyl stehen, wobei zwei benachbarte Reste R auch jeweils gemeinsam einen cyclischen Rest wie Cyclopentyl, Cyclohexyl oder 1,2-Phenylen bilden können.

Als typische Kronenether der Formel (VI) seien beispielhaft genannt:
Benzo-15-krone-5, 15-Krone-5, 18-Krone-6, Dibenzo-18-krone-6, Dibenzo-24-krone-8 und Dicyclohexano-18-krone-6.

Bevorzugt verwendet man 18-Krone-6, Dibenzo-18-krone-6 und Dibenzo-24-krone-8.

Besonders bevorzugt ist 18-Krone-6.

Die Menge an Phasentransferkatalysator im erfindungsgemäßen Verfahren liegt zwischen 1 und 100 Molprozent, bezogen auf Arylhalogenid der Formel (I). Bevorzugt sind Mengen zwischen 25 und 75 Molprozent.

Das erfindungsgemäße Verfahren wird bei Temperaturen von 0 °C bis 220 °C, vorzugsweise bei 50 °C bis 200 °C und besonders bevorzugt bei 100°C bis 180 °C durchgeführt.

Das erfindungsgemäße Verfahren kann in Gegenwart eines Lösungsmittels oder unter Verwendung eines Überschusses an Malonester der Formel (II) durchgeführt werden. Vorzugsweise wird in Gegenwart eines Überschusses an Malonester der Formel (II) gearbeitet.

Der Überschuß an Malonester der Formel (II) liegt zwischen 2 und 20 Moläquivalenten, bezogen auf das Arylhalogenid der Formel (I). Bevorzugt arbeitet man mit Überschüssen von 3 bis 10 Moläquivalenten.

Das erfindungsgemäße Verfahren wird üblicherweise bei Normaldruck durchgeführt, kann aber auch bei verringertem oder erhöhtem Druck durchgeführt werden.

Zur Isolierung der erfindungsgemäß hergestellten Aryl- und Heteroarylessigsäuren und ihrer Derivate wird das Reaktionsgemisch nach Beendigung der Reaktion vorzugsweise destillativ und/oder durch Extraktion oder chromatographische Methoden aufgearbeitet.

Das erfindungsgemäße Verfahren wird durch die folgenden Beispiele veranschaulicht, ohne auf diese eingeschränkt zu sein.

### Herstellungsbeispiele

### Beispiel 1: 4-Methylphenylessigsäureethylester

In einem trockenem Schlenk-Gefäß wurden 171 mg [1 mmol] 4-Bromtoluol, 1056 mg [6,6 mmol] Malonsäurediethylester, 2,88 mg [0,005 mmol] Pd(dba)₂, 3,19 mg [0,011 mmol] P(tert-Bu)₃ x HBF₄, 594 mg [2,8 mmol] getrocknetes K₃PO₄ und 132 mg [0,5 mmol] 18-Krone-6 vorgelegt. Das Reaktionsgefäß wurde dreimal evakuiert und mit Stickstoff befüllt. Dann wurde bis zum vollständigen Umsatz (8 bis 12 Stunden) bei 160°C gerührt. Nach Abkühlen auf Raumtemperatur wurde das Reaktionsgemisch mit Essigester verdünnt. Die resultierende Lösung wurde nacheinander mit je 20 ml Wasser, gesättigter wssg. NaHCO₃-Lsg. und gesättigter wssg. NaCl-Lösung gewaschen, über MgSO₄ getrocknet, filtriert und im Vakuum eingeengt. Chromatographische Reinigung über Kieselgel (Hexan/ Essigester) lieferte 4-Methylphenylessigsäureethylester in einer Ausbeute von 88% der Theorie.
¹H-NMR (400 MHz, CDCl₃): δ = 7.19 (d, *J* = 8.0 Hz, 2H), 7.14 (d, *J* = 8.0 Hz, 2H), 4.16 (q, *J* = 8.0 Hz, 2H), 3.58 (s, 2H), 2.34 (s, 3H), 1.26 (t, *J* = 8.0 Hz, 3H). ¹³C-NMR (101 MHz, CDCl₃): δ = 171.8, 136.6, 131.1, 129.2, 129.1, 60.8, 41.0, 21.0, 14.2. MS (70 eV), m/z (%): 178 (34) [M⁺], 106 (10), 105 (100). IR (NaCl): *ν̃* = 2980 (vs), 2927 (m), 1735 (vs), 1515 (m), 1446 (m), 1367 (m), 1301 (m), 1253 (m), 1152 (m), 1032 (m), 809 (m).

### Beispiel 2: 2-Ethylphenylessigsäureethylester

Analog zu Beispiel 1 wurden aus 185 mg [1 mmol] 2-Ethyl-brombenzol 170 mg der Titelverbindung erhalten (88% der Theorie).
¹H-NMR (400 MHz, CDCl₃): δ = 7.27-7.16 (m, 4H), 7.56 (t, *J* = 8.0 Hz, 1H), 7.46 (t, *J* = 8.0 Hz, 2H), 7.39 (d, *J* = 8.0 Hz, 2H), 4.17 (q, *J* = 8.0 Hz, 2H), 3.68 (s, 2H), 2.70 (q, *J* = 8.0 Hz, 2H), 1.29-1.23 (m, 6H). ¹³C-NMR (101 MHz, CDCl₃): δ = 171.6, 142.5, 132.0, 130.3, 128.4, 127.4, 125.9, 60.7, 38.5, 25.7, 14.8, 14.1. MS (70 eV), m/z (%): 193 (4), 192 (24) [M⁺], 146 (29), 119 (100), 91 (54), 77 (21). IR (NaCl): *ṽ* = 2980 (vs), 2935 (m), 1734 (vs), 1615 (m), 1583 (w), 1513 (s), 1246 (s), 1032 (m), 821 (m).

### Beispiel 3: 3-Methoxyphenylessigsäureethylester

Analog zu Beispiel 1 wurden aus 187 mg [1 mmol] 3-Bromanisol 180 mg der Titelverbindung erhalten (93% der Theorie).
¹H-NMR (400 MHz, CDCl₃): δ = 7.25 (t, *J* = 8.0 Hz, 1H), 6.91-6.81 (m, 3H), 4.17 (q, *J* = 8.0 Hz, 2H), 3,81 (s, 3H), 3.60 (s, 2H), 1.27 (t, *J* = 8.0 Hz, 3H). ¹³C-NMR (101 MHz, CDCl₃): δ = 171.4, 159.6, 135.5, 129.4, 121.5, 114.8, 112.5, 60.8, 55.1, 41.4, 14.1. MS (70 eV), m/z (%): 195 (7), 194 (50) [M⁺], 121 (100), 91 (37), 78 (17), 77 (26). IR (NaCl): *ν̃* = 2979 (vs), 1731 (vs), 1601 (s), 1586 (m), 1492 (m), 1368 (m), 1262 (m), 1031 (m), 870 (m), 773 (m).

### Beispiel 4: 4-Methylthiophenylessigsäureethylester

Analog zu Beispiel 1 wurden aus 203 mg [1 mmol] 4-Brom-thioanisol 199 mg der Titelverbindung erhalten (95% der Theorie).
¹H-NMR (400 MHz, CDCl₃): δ = 7.25-7.18 (m, 4H), 4.14 (q, *J* = 8.0 Hz, 2H), 3.56 (s, 2H), 2.46 (s, 3H), 1.24 (t, *J* = 8.0 Hz, 3H). ¹³C-NMR (101 MHz, CDCl₃): δ = 171.5, 137.1, 131.0, 129.7, 126.9, 60.7, 40.8, 16.0, 14.2. MS (70 eV), m/z (%): 211 (16), 210 (100) [M⁺], 137 (88), 121 (9). IR (KBr): *ṽ* = 1730 (vs), 1495 (m), 1469 (m), 1366 (m), 1225 (m), 1031 (m), 802 (s).

### Beispiel 5: 3-Thienyl-essigsäureethylester

Analog zu Beispiel 1 wurden aus 163 mg [1 mmol] 3-Brom-thiophen 160 mg der Titelverbindung erhalten (94% der Theorie).
¹H-NMR (400 MHz, CDCl₃): δ = 7.29-7.26 (m, 1H), 7.14 (s, 1H), 7.04 (d, *J* = 8.0 Hz, 1H), 4.16 (q, *J* = 8.0 Hz, 2H), 3.64 (s, 2H), 1.26 (t, *J* = 8.0 Hz, 3H). ¹³C-NMR (101 MHz, CDCl₃): δ = 170.1, 133.7, 128.5, 125.6, 122.7, 60.9, 35.9, 14.1. MS (70 eV), m/z (%): 171 (10), 170 (58) [M⁺], 98 (22), 97 (100). R (NaCl): *ν̃* = 2979 (s), 2937 (m), 1733 (vs), 1464 (m), 1369 (m), 1259 (m), 1206 (m), 1155 (m), 1028 (m).

### Beispiel 6: 4-Methylphenylessigsäureethylester

Analog zu Beispiel 1 wurde aus 128 mg [1 mmol] 4-Chlortoluol die Titelverbindung in einer Ausbeute von 85% der Theorie erhalten.

### Beispiel 7: 4-Methylphenylessigsäureethylester

Analog zu Beispiel 1 wurde aus 218 mg [1 mmol] 4-Iodtoluol die Titelverbindung in einer Ausbeute von 91% der Theorie erhalten.

### Beispiel 8: 2-Naphthylessigsäureethylester

Analog zu Beispiel 1 wurden aus 207 mg [1 mmol] 2-Brom-naphthalin 200 mg der Titelverbindung erhalten (93% der Theorie).
¹H-NMR (400 MHz, CDCl₃): δ = 7.87-7.79 (m, 3H), 7.75 (s, 1H), 7.51-7.42 (m, 3H), 4.18 (q, *J* = 8.0 Hz, 2H), 3.79 (s, 2H), 1.27 (t, *J* = 8.0 Hz, 3H). ¹³C-NMR (101 MHz, CDCl₃): δ = 171.5, 133.4, 132.4, 131.6, 128.1, 127.9, 127.61, 127.58, 127.3, 126.0, 125.7, 60.9, 41.6, 14.2. MS (70 eV), m/z (%): 215 (10), 214 (57) [M⁺], 141 (100), 115 (31). IR (NaCl): *ṽ* = 2980 (vs), 2936 (m), 1734 (vs), 1601 (m), 1508 (m), 1368 (m), 1258 (m), 1159 (m), 1031 (s), 859 (m), 818 (m), 802 (m). 759 (m), 742 (m).

### Beispiel 9: 4-Methylphenylessigsäureethylester

In einem trockenem Schlenk-Gefäß wurden 171 mg [1 mmol] 4-Bromtoluol, 1056 mg [6,6 mmol] Malonsäurediethylester, 1,12 mg [0,005 mmol] Pd(OAc)₂, 3,19 mg [0,011 mmol] P(tert-Bu)₃ x HBF₄, 594 mg [2,8 mmol] getrocknetes K₃PO₄ und 132 mg [0,5 mmol] 18-Krone-6 vorgelegt. Das Reaktionsgefäß wurde dreimal evakuiert und mit Stickstoff befüllt. Dann wurde bis zum vollständigen Umsatz (8 bis 12 Stunden) bei 160°C gerührt. Nach Abkühlen auf Raumtemperatur wurde das Reaktionsgemisch mit Essigester verdünnt. Die resultierende Lösung wurde nacheinander mit je 20 ml Wasser, gesättigter wssg. NaHCO₃-Lsg. und gesättigter wssg. NaCl-Lösung gewaschen, über MgSO₄ getrocknet, filtriert und im Vakuum eingeengt. Chromatographische Reinigung über Kieselgel (Hexan/ Essigester) lieferte 4-Methylphenylessigsäureethylester in einer Ausbeute von 75% der Theorie.

### Beispiel 10: 4-Methylphenylessigsäureethylester

In einem trockenem Schlenk-Gefäß wurden 171 mg [1 mmol] 4-Bromtoluol, 1056 mg [6,6 mmol] Malonsäurediethylester, 2,88 mg [0,005 mmol] Pd(dba)₂, 2,22 mg [0,011 mmol] P(tert-Bu)₃, 594 mg [2,8 mmol] getrocknetes K₃PO₄ und 132 mg [0,5 mmol] 18-Krone-6 vorgelegt. Das Reaktionsgefäß wurde dreimal evakuiert und mit Stickstoff befüllt. Dann wurde bis zum vollständigen Umsatz (8 bis 12 Stunden) bei 160°C gerührt. Nach Abkühlen auf Raumtemperatur wurde das Reaktionsgemisch mit Essigester verdünnt. Die resultierende Lösung wurde nacheinander mit je 20 ml Wasser, gesättigter wssg. NaHCO₃-Lsg. und gesättigter wssg. NaCl-Lösung gewaschen, über MgSO₄ getrocknet, filtriert und im Vakuum eingeengt. Chromatographische Reinigung über Kieselgel (Hexan/ Essigester) lieferte 4-Methylphenylessigsäureethylester in einer Ausbeute von 76% der Theorie.

## Patentansprüche

1. Verfahren zur Herstellung von Verbindungen der Formel (III) 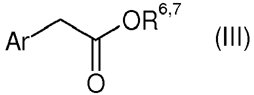 in welcher
Ar für die Gruppe 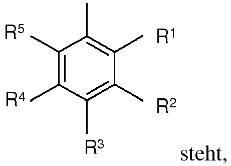 wobei
R¹, R², R³, R⁴ und R⁵ unabhängig voneinander gleich oder verschieden für Wasserstoff, Amino, Cyano, Nitro, Halogen, für gegebenenfalls durch Halogen substituiertes C₁-C₆-Alkyl, C₁-C₆-Thioalkyl, Thiophenyl, C₁-C₆-Alkoxy, C₆-C₁₀-Aryloxy, Phenyl, -CO-C₆-C₁₀-Aryl, - CO-C₁-C₃-Alkyl, -COO-C₁-C₆-Alkyl oder -COO-C₆-C₁₀-Aryl stehen,
der Rest Ar kann darüber hinaus auch für einen heteroaromatischen Rest stehen oder
der Rest Ar kann auch für 1- oder 2-Naphthyl stehen,
und
R⁶ und R⁷ unabhänging voneinander für gegebenenfalls substituiertes C₁-C₈-Alkyl, Phenyl, Aryl, oder für NR⁸R⁹ stehen,
wobei R⁸ und R⁹ unabhängig voneinander gleich oder verschieden für C₁-C₄-Alkyl oder für gegebenenfalls durch C₁-C₃-Alkyl, welches durch Fluor oder Chlor substituiert sein kann, durch Nitro, Cyano oder Di-C₁-C₃-Alkylamino substituiertes Phenyl stehen, oder gemeinsam mit dem Stickstoffatom, an das sie gebunden sind, für einen gesättigten oder ungesättigten, substituierten oder unsubstituierten Cyclus stehen,
**dadurch gekennzeichnet, dass** man Aryl- oder Heteroarylhalogenide der Formel (I)
Ar-Hal (I)
in welcher
Hal für Chlor, Brom oder Iod steht und
Ar die oben angegebenen Bedeutungen hat,
mit Malonsäureestern der Formel (II) 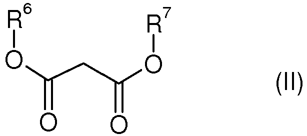 in welcher
R⁶ und R⁷ die oben angegebenen Bedeutungen haben
in Gegenwart eines Palladiumkatalysators, eines Phosphin-Liganden und
A) einer anorganischen Base und eines Phasentransferkatalysators
gegebenenfalls unter Verwendung eines organischen Lösungsmittels umsetzt.

2. Verfahren zur Herstellung von Verbindungen der Formel (III) gemäß Anspruch 1, wobei
Ar für 1- oder 2-Naphthyl, 3-Thienyl oder für die Gruppe 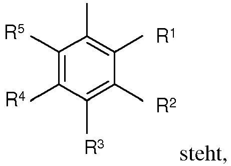 wobei
R¹, R², R³, R⁴ und R⁵ unabhängig voneinander gleich oder verschieden für Wasserstoff, Amino, Cyano, Nitro, Fluor, für gegebenenfalls durch Fluor substituiertes C₁-C₄-Alkyl, C₁-C₄-Thioalkyl, Thiophenyl, C₁-C₄-Alkoxy, C₆-C₁₀-Aryloxy, Phenyl, -CO-C₆-C₈-Aryl, -CO-C₁-C₃-Alkyl, -COO-C₁-C₄-Alkyl oder -COO-C₆-C₈-Aryl stehen,
Hal für Chlor, Brom oder Iod steht,
R⁶ und R⁷ unabhängig voneinander gleich oder verschieden für C₁-C₄-Alkyl stehen.

3. Verfahren zur Herstellung von Verbindungen der Formel (III) gemäß Anspruch 1, wobei
Ar für 1-oder 2-Naphthyl, 3-Thienyl oder für die Gruppe 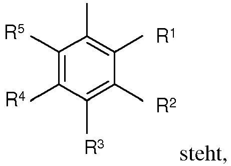 wobei
R¹, R², R³, R⁴ und R⁵ unabhängig voneinander gleich oder verschieden für Wasserstoff, Amino, Cyano, Nitro, Fluor, Methyl, Methylthio, Ethyl, i-Propyl, n-Propyl, CF₃, C₂F₅, C₃F₇, Methoxy, Ethoxy, Phenyl, -CO-Phenyl, -CO-Methyl, -CO-Ethyl, -COO-Methyl, -COO-Ethyl oder -COO-Phenyl stehen,
Hal für Chlor, Brom oder Iod steht,
R⁶ und R⁷ unabhängig voneinander für Methyl oder Ethyl stehen.

4. Verfahren zur Herstellung von Verbindungen der Formel (III) gemäß Anspruch 1, wobei
Ar für 1-Naphthyl, 2-Naphthyl, Phenyl, 4-N,N-Dimethylaminophenyl, 4-Methylthiophenyl, 4-Methoxyphenyl, 4-Ethoxyphenyl, 3-Methoxyphenyl, 2-Methoxyphenyl, 2-Methylphenyl, 3-Methylphenyl, 4-Methylphenyl, 4-Fluorphenyl, 3-Fluorphenyl, 2-Fluorphenyl, 2-Ethylphenyl, 4-Ethoxycarbonylphenyl, 3-Thienyl steht.

5. Verfahren zur Herstellung von Verbindungen der Formel (III) gemäß Anspruch 1, wobei Ar für 2,6-Dimethylphenyl, 2,4,6-Trimethylphenyl, 4-Cyanophenyl, 4-Cyano-2-methylphenyl, 3-Cyanophenyl, 4-Ethoxycarbonylphenyl, 4-Trifluormethylphenyl, 4-Acetylphenyl, 4-Nitrophenyl, 4-Benzoylphenyl steht.

6. Verfahren zur Herstellung von Verbindungen der Formel (III) gemäß Anspruch 1, wobei R⁶ und R⁷ für Ethyl stehen.

7. Verfahren zur Herstellung von Verbindungen der Formel (III) gemäß Anspruch 1 **dadurch gekennzeichnet, dass** als Palladiumkatalysatoren Bis(dibenzylidenaceton)palladium, Tris(di-benzylidenaceton)dipalladium oder Palladiumacetat eingesetzt werden.

8. Verfahren zur Herstellung von Verbindungen der Formel (III) gemäß Anspruch 1 **dadurch gekennzeichnet, dass** als Phosphin-Ligand Tri-tert-butyl-phosphin, Tricyclohexylphosphin, Tris(1-adamantyl)phosphin, n-Butyl-di(1-adamantyl)-phosphin (cataCXium® A), Benzyl-di(1-adamantyl)-phosphin (cataCXium® ABn), 2-(Di-tert-butylphosphino)biphenyl (JohnPhos) oder 2-(Di-cyclohexylphosphino)-2'-(N,N-dimethylamino) eingesetzt werden.

9. Verfahren zur Herstellung von Verbindungen der Formel (III) gemäß Anspruch 1 **dadurch gekennzeichnet, dass** als Phosphin-Ligand Tri-tert.butyl-phosphin eingesetzt wird.

10. Verfahren zur Herstellung von Verbindungen der Formel (III) gemäß Anspruch 1 **dadurch gekennzeichnet, dass** im Verfahrensschritt A) als Base Kaliumphosphat eingesetzt wird.

11. Verfahren zur Herstellung von Verbindungen der Formel (III) gemäß Anspruch 1, **dadurch gekennzeichnet, dass** als Phasentransferkatalysatoren quaternären Ammoniumsalze oder Phosphoniumsalze der Formel (V) 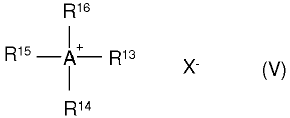 in welcher
R¹³, R¹⁴, R¹⁵ und R¹⁶ unabhängig voneinander gleich oder verschieden für C₁-C₂₈-Alkyl, gegebenenfalls verzweigtes C₁-C₂₈-Alkyl, C₆-C₁₀-Aryl, oder Benzyl stehen,
A für N oder P steht und
X für Halogen, Hydrogensulfat, Sulfat, Dihydrogenphosphat, Hydrogenphosphat, Phosphat oder Acetat steht eingesetzt werden.

12. Verfahren zur Herstellung von Verbindungen der Formel (III) gemäß Anspruch 1, **dadurch gekennzeichnet, dass** als Phasentransferkatalysatoren Kronenether der Formel (VI) 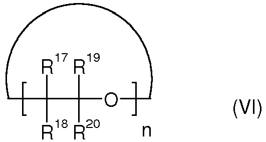 in der n für eine Zahl zwischen 4 und 8 steht
und die Reste R¹⁷ bis R²⁰ unabhängig voneinander für Wasserstoff, C₁-C₄-Alkyl oder Phenyl stehen, wobei zwei benachbarte Reste R auch jeweils gemeinsam einen cyclischen Rest wie Cyclopentyl, Cyclohexyl oder 1,2-Phenylen bilden können,
eingesetzt werden.

13. Verfahren zur Herstellung von Verbindungen der Formel (III) gemäß Anspruch 1, **dadurch gekennzeichnet, dass** als Phasentransferkatalysator 18-Krone-6 eingesetzt wird.

14. Verfahren zur Herstellung von Verbindungen der Formel (III) gemäß Anspruch 1, **dadurch gekennzeichnet, dass** Malonester der Formel (II) als Lösungsmittel im Überschuss verwendet wird.

15. Verfahren zur Herstellung von Verbindungen der Formel (III) gemäß Anspruch 1, **dadurch gekennzeichnet, dass** bei Temperaturen von 100 bis 180 °C gearbeitet wird.

## Claims

1. Process for preparing compounds of the formula (III) 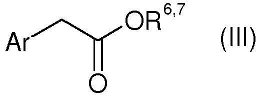 in which
Ar is the 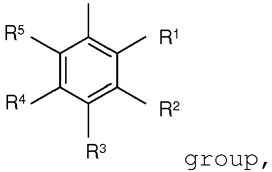 where
R¹, R², R³, R⁴ and R⁵ are the same or different and are each independently hydrogen, amino, cyano, nitro, halogen, optionally halogen-substituted C₁-C₆-alkyl, C₁-C₆-thioalkyl, thiophenyl, C₁-C₆-alkoxy, C₆-C₁₀-aryloxy, phenyl, -CO-C₆-C₁₀-aryl, -CO-C₁-C₃-alkyl, -COO-C₁-C₆-alkyl or -COO-C₆-C₁₀-aryl,
the Ar radical may additionally also be a heteroaromatic radical or
the Ar radical may also be 1- or 2-naphthyl,
and
R⁶ and R⁷ are each independently optionally substituted C₁-C₈-alkyl, phenyl, aryl, or NR⁸R⁹,
where R⁸ and R⁹ are the same or different and are each independently C₁-C₄-alkyl, or phenyl optionally substituted by optionally fluorine- or chlorine-substituted C₁-C₃-alkyl, by nitro, cyano or di-C₁-C₃-alkylamino, or together with the nitrogen atom to which they are bonded are a saturated or unsaturated, substituted or unsubstituted cycle,
**characterized in that** aryl or heteroaryl halides of the formula (I)
Ar-Hal (I)
in which
Hal is chlorine, bromine or iodine and
Ar is as defined above,
are reacted with malonic esters of the formula (II) 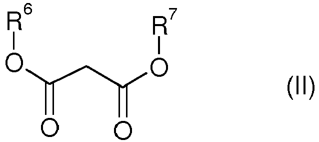 in which
R⁶ and R⁷ are each as defined above
in the presence of a palladium catalyst, of a phosphine ligand and
A) of an inorganic base and of a phase transfer catalyst
optionally using an organic solvent.

2. Process for preparing compounds of the formula (III) according to Claim 1 where
Ar is 1- or 2-naphthyl, 3-thienyl or the 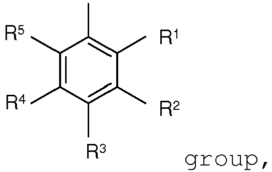 where
R¹, R², R³, R⁴ and R⁵ are the same or different and are each independently hydrogen, amino, cyano, nitro, fluorine, optionally fluorine-substituted C₁-C₄-alkyl, C₁-C₄-thioalkyl, thiophenyl, C₁-C₄-alkoxy, C₆-C₁₀-aryloxy, phenyl, -CO-C₆-C₈-aryl, -CO-C₁-C₃-alkyl, -COO-C₁-C₄-alkyl or -COO-C₆-C₈-aryl,
Hal is chlorine, bromine or iodine,
R⁶ and R⁷ are the same or different and are each independently C₁-C₄-alkyl.

3. Process for preparing compounds of the formula (III) according to Claim 1 where
Ar is 1- or 2-naphthyl, 3-thienyl or the 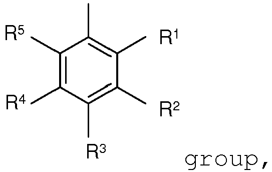 where
R¹, R², R³, R⁴ and R⁵ are the same or different and are each independently hydrogen, amino, cyano, nitro, fluorine, methyl, methylthio, ethyl, i-propyl, n-propyl, CF₃, C₂F₅, C₃F₇, methoxy, ethoxy, phenyl, -CO-phenyl, -CO-methyl, -CO-ethyl, -COO-methyl, -COO-ethyl or -COO-phenyl,
Hal is chlorine, bromine or iodine,
R⁶ and R⁷ are each independently methyl or ethyl.

4. Process for preparing compounds of the formula (III) according to Claim 1 where
Ar is 1-naphthyl, 2-naphthyl, phenyl, 4-N,N-dimethylaminophenyl, 4-methylthiophenyl, 4-methoxyphenyl, 4-ethoxyphenyl, 3-methoxyphenyl, 2-methoxyphenyl, 2-methylphenyl, 3-methylphenyl, 4-methylphenyl, 4-fluorophenyl, 3-fluorophenyl, 2-fluorophenyl, 2-ethylphenyl, 4-ethoxycarbonylphenyl, 3-thienyl.

5. Process for preparing compounds of the formula (III) according to Claim 1 where
Ar is 2,6-dimethylphenyl, 2,4,6-trimethylphenyl, 4-cyanophenyl, 4-cyano-2-methylphenyl, 3-cyanophenyl, 4-ethoxycarbonylphenyl, 4-trifluoromethylphenyl, 4-acetylphenyl, 4-nitrophenyl, 4-benzoylphenyl.

6. Process for preparing compounds of the formula (III) according to Claim 1 where
R⁶ and R⁷ are each ethyl.

7. Process for preparing compounds of the formula (III) according to Claim 1, **characterized in that** the palladium catalyst used is bis(dibenzylideneacetone)palladium, tris(dibenzylideneacetone)dipalladium or palladium acetate.

8. Process for preparing compounds of the formula (III) according to Claim 1, **characterized in that** the phosphine ligand used is tri-tert-butylphosphine, tricyclohexylphosphine, tris(1-adamantyl)phosphine, n-butyldi(1-adamantyl)phosphine (cataCXium® A), benzyldi(1-adamantyl)phosphine (cataCXium® ABn), 2-(di-tert-butylphosphino)biphenyl (JohnPhos) or 2-(dicyclohexylphosphino)-2'-(N,N-dimethylamino.

9. Process for preparing compounds of the formula (III) according to Claim 1, **characterized in that** the phosphine ligand used is tri(tert-butyl)phosphine.

10. Process for preparing compounds of the formula (III) according to Claim 1, **characterized in that** the base used in process step A) is potassium phosphate.

11. Process for preparing compounds of the formula (III) according to Claim 1, **characterized in that** the phase transfer catalysts used are quaternary ammonium salts or phosphonium salts of the formula (V) 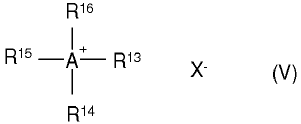 in which
R¹³, R¹⁴, R¹⁵ and R¹⁶ are the same or different and are each independently C₁-C₂₈-alkyl, optionally branched C₁-C₂₈-alkyl, C₆-C₁₀-aryl, or benzyl,
A is N or P and
X is halogen, hydrogensulphate, sulphate, dihydrogenphosphate, hydrogenphosphate, phosphate or acetate.

12. Process for preparing compounds of the formula (III) according to Claim 1, **characterized in that** the phase transfer catalysts used are crown ethers of the formula (VI) 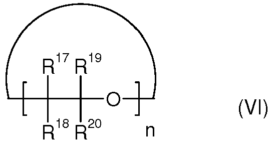 in which n is a number from 4 to 8
and the R¹⁷ to R²⁰ radicals are each independently hydrogen, C₁-C₄-alkyl or phenyl, where two adjacent R radicals may also in each case together form a cyclic radical such as cyclopentyl, cyclohexyl or 1,2-phenylene.

13. Process for preparing compounds of the formula (III) according to Claim 1, **characterized in that** the phase transfer catalyst used is 18-crown-6.

14. Process for preparing compounds of the formula (III) according to Claim 1, **characterized in that** malonic ester of the formula (II) is used as a solvent in excess.

15. Process for preparing compounds of the formula (III) according to Claim 1, **characterized in that** temperatures of 100 to 180°C are employed.

## Revendications

1. Procédé de fabrication de composés de formule (III) 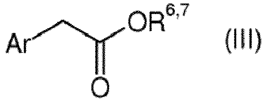 dans laquelle
Ar représente le groupe 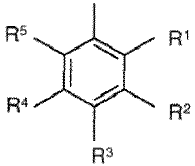
R¹, R², R³, R⁴ et R⁵ représentant indépendamment les uns des autres, de manière identique ou différente, hydrogène, amino, cyano, nitro, halogène, alkyle en C₁-C₆ éventuellement substitué par halogène, thioalkyle en C₁-C₆, thiophényle, alcoxy en C₁-C₆, aryloxy en C₆-C₁₀, phényle, -CO-aryle en C₆-C₁₀, -CO-alkyle en C₁-C₃, -COO-alkyle en C₁-C₆ ou -COO-aryle en C₆-C₁₀,
le radical Ar peut en outre également représenter un radical hétéroaromatique, ou
le radical Ar peut également représenter 1- ou 2-naphtyle,
et
R⁶ et R⁷ représentent indépendamment l'un de l'autre alkyle en C₁-C₈ éventuellement substitué, phényle, aryle ou NR⁸R⁹,
R⁸ et R⁹ représentant indépendamment l'un de l'autre, de manière identique ou différente, alkyle en C₁-C₄ ; ou phényle éventuellement substitué par alkyle en C₁-C₃, qui peut être substitué par fluor ou chlore, par nitro, cyano ou di-alkylamino en C₁-C₃, ou représentent conjointement avec l'atome d'azote auquel ils sont reliés un cycle saturé ou insaturé, substitué ou non substitué,
**caractérisé en ce que** des halogénures d'aryle ou d'hétéroaryle de formule (I)
Ar-Hal (I)
dans laquelle
Hal représente chlore, brome ou iode, et
Ar a les significations indiquées précédemment,
sont mis en réaction avec des esters de l'acide malonique de formule (II) 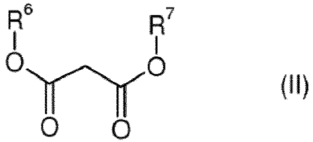 dans laquelle
R⁶ et R⁷ ont les significations indiquées précédemment, en présence d'un catalyseur de palladium, d'un ligand phosphine et
A) d'une base inorganique et d'un catalyseur de transfert de phase,
éventuellement en utilisant un solvant organique.

2. Procédé de fabrication de composés de formule (III) selon la revendication 1, dans lequel
Ar représente 1- ou 2-naphtyle, 3-thiényle, ou le groupe 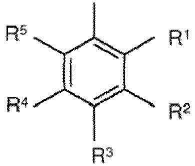
R¹, R², R³, R⁴ et R⁵ représentant indépendamment les uns des autres, de manière identique ou différente, hydrogène, amino, cyano, nitro, fluor, alkyle en C₁-C₄ éventuellement substitué par fluor, thioalkyle en C₁-C₄, thiophényle, alcoxy en C₁-C₄, aryloxy en C₆-C₁₀, phényle, -CO-aryle en C₆-C₈, -CO-alkyle en C₁-C₃, -COO-alkyle en C₁-C₄ ou -COO-aryle en C₆-C₈,
Hal représente chlore, brome ou iode,
R⁶ et R⁷ représentent indépendamment l'un de l'autre, de manière identique ou différente, alkyle en C₁-C₄.

3. Procédé de fabrication de composés de formule (III) selon la revendication 1, dans lequel Ar représente 1- ou 2-naphtyle, 3-thiényle, ou le groupe 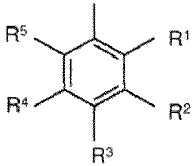
R¹, R², R³, R⁴ et R⁵ représentant indépendamment les uns des autres, de manière identique ou différente, hydrogène, amino, cyano, nitro, fluor, méthyle, méthylthio, éthyle, i-propyle, n-propyle, CF₃, C₂F₅, C₃F₇, méthoxy, éthoxy, phényle, -CO-phényle, -CO-méthyle, -CO-éthyle, -COO-méthyle, -COO-éthyle ou -COO-phényle,
Hal représente chlore, brome ou iode,
R⁶ et R⁷ représentent indépendamment l'un de l'autre méthyle ou éthyle.

4. Procédé de fabrication de composés de formule (III) selon la revendication 1, dans lequel Ar représente 1-naphtyle, 2-naphtyle, phényle, 4-N,N-diméthylaminophényle, 4-méthylthiophényle, 4-méthoxyphényle, 4-éthoxyphényle, 3-méthoxyphényle, 2-méthoxyphényle, 2-méthylphényle, 3-méthylphényle, 4-méthylphényle, 4-fluorophényle, 3-fluorophényle, 2-fluorophényle, 2-éthylphényle, 4-éthoxycarbonylphényle, 3-thiényle.

5. Procédé de fabrication de composés de formule (III) selon la revendication 1, dans lequel Ar représente 2,6-diméthylphényle, 2,4,6-triméthylphényle, 4-cyanophényle, 4-cyano-2-méthylphényle, 3-cyanophényle, 4-éthoxycarbonylphényle, 4-trifluorométhylphényle, 4-acétylphényle, 4-nitrophényle, 4-benzoylphényle.

6. Procédé de fabrication de composés de formule (III) selon la revendication 1, dans lequel R⁶ et R⁷ représentent éthyle.

7. Procédé de fabrication de composés de formule (III) selon la revendication 1, **caractérisé en ce que** du bis(dibenzylidène-acétone)palladium, du tris(dibenzylidène-acétone)dipalladium ou de l'acétate de palladium est utilisé en tant que catalyseur de palladium.

8. Procédé de fabrication de composés de formule (III) selon la revendication 1, **caractérisé en ce que** de la tri-tert-butyl-phosphine, de la tricyclohexylphosphine, de la tris(1-adamantyl)phosphine, de la n-butyl-di(1-adamantyl)-phosphine (cataCXium® A), de la benzyl-di(1-adamantyl)-phosphine (cataCXium® ABn), du 2-(di-tert-butylphosphino)biphényl (JohnPhos) ou du 2-(di-cyclohexylphosphino)-2'-(N,N-diméthylamino) est utilisé en tant que ligand phosphine.

9. Procédé de fabrication de composés de formule (III) selon la revendication 1, **caractérisé en ce que** de la tri-tert.-butyl-phosphine est utilisée en tant que ligand phosphine.

10. Procédé de fabrication de composés de formule (III) selon la revendication 1, **caractérisé en ce que** du phosphate de potassium est utilisé en tant que base à l'étape de procédé A).

11. Procédé de fabrication de composés de formule (III) selon la revendication 1, **caractérisé en ce que** des sels d'ammonium quaternaires ou des sels de phosphonium de formule (V) sont utilisés en tant que catalyseurs de transfert de phase 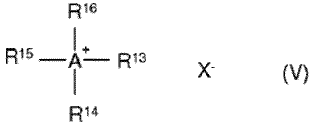 dans laquelle
R¹³, R¹⁴, R¹⁵ et R¹⁶ représentent indépendamment les uns des autres, de manière identique ou différente, alkyle en C₁-C₂₈, alkyle en C₁-C₂₈ éventuellement ramifié, aryle en C₆-C₁₀ ou benzyle,
A représente N ou P, et
X représente halogène, hydrogénosulfate, sulfate, dihydrogénophosphate, hydrogénophosphate, phosphate ou acétate.

12. Procédé de fabrication de composés de formule (III) selon la revendication 1, **caractérisé en ce que** des éthers couronnes de formule (VI) sont utilisés en tant que catalyseurs de transfert de phase 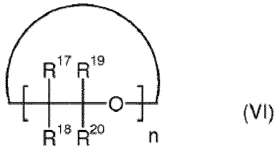 dans laquelle n représente un nombre compris entre 4 et 8,
et les radicaux R¹⁷ à R²⁰ représentent indépendamment les uns des autres hydrogène, alkyle en C₁-C₄ ou phényle, deux radicaux R voisins pouvant également former ensemble un radical cyclique tel que cyclopentyle, cyclohexyle ou 1,2-phénylène.

13. Procédé de fabrication de composés de formule (III) selon la revendication 1, **caractérisé en ce que** du 18-couronne-6 est utilisé en tant que catalyseur de transfert de phase.

14. Procédé de fabrication de composés de formule (III) selon la revendication 1, **caractérisé en ce que** des esters maloniques de formule (II) sont utilisés en excès en tant que solvant.

15. Procédé de fabrication de composés de formule (III) selon la revendication 1, **caractérisé en ce qu'**il est réalisé à des températures de 100 à 180 °C.
